# EUROPEAN PATENT APPLICATION

(11) **EP 4 671 358 A1**
(43) Date of publication of application: **31.12.2025**
(21) Application number: 24866801.4
(22) Date of filing: 22.03.2024
(51) Int. Cl.: C12N 1/10, C05F 11/08, C05G 3/80, A01N 63/28, A01P 21/00, C12R 1/465

(54) **STREPTOMYCES, FERMENTATION BROTH, AND USE OF BOTH**

(30) Priority: 19.09.2023 CN 202311211459
(71) Applicant: Sinofert Holdings Limited Linyi Agriculture R&D Center, Linyi, Shandong 276000 (CN); Sinochem Agriculture Linyi R&D Center Co., Ltd., Linyi, Shandong 276000 (CN)
(72) Inventor: LIU, Wei, Linyi, Shandong 276000 (CN); REN, Xianshun, Linyi, Shandong 276000 (CN); ZHAO, Jianyu, Linyi, Shandong 276000 (CN); ZHOU, Jiachao, Linyi, Shandong 276000 (CN); MAN, Liping, Linyi, Shandong 276000 (CN); ZHANG, Shuai, Linyi, Shandong 276000 (CN)
(74) Representative: Haseltine Lake Kempner LLP
(86) International application number: PCT/CN2024/083318
(87) International publication number: WO 2025/060374

(57) **Abstract**

Provided are Streptomyces and a use thereof. The Streptomyces is named *Streptomyces linyiensis*, which was preserved in China General Microbiological Culture Collection Center (CGMCC) on September 28, 2022, and has a preservation number of CGMCC No. 25834. A Streptomyces fermentation broth can be used to significantly improve the abundance of nitrogen-fixing microorganisms in crop rhizosphere soil, improve the nitrogen-fixing enzyme activity of the crop rhizosphere soil, improve the nitrogen-fixing capability of the nitrogen-fixing microorganisms in the crop rhizosphere soil, and promote the absorption of nitrogen nutrients by crops, and can be used for preparing bacterial agents, microbial fertilizers, or biopesticides having corresponding functions.

## Description

### PRIORITY INFORMATION

The present disclosure claims priority and benefits of the patent application No. 202311211459.1, filed with the China National Intellectual Property Administration on September 19, 2023, which is incorporated herein by reference in its entirety.

### FIELD

The present disclosure relates to the field of microbial technology, and more specifically, to a Streptomyces which can increase the abundance of the nitrogen-fixing microorganism in the rhizosphere soil of crops, increase the nitrogenase activity in the rhizosphere soil of the crops, increase the nitrogen-fixing ability of the nitrogen-fixing microorganism in the rhizosphere soil of the crops, and promote the absorption of nitrogen nutrients by the crops, and use thereof.

### BACKGROUND

Soil microorganisms are a general term for bacteria, fungi, actinomycetes, and algae that live in the soil. Their individuals are tiny, generally measured in micrometers or millimeter, and there are usually 10⁶ to 10⁹ of them in 1 gram of soil. Their types and numbers vary with the soil-forming environment and the depth of the soil layer. They carry out oxidation, nitrification, ammoniation, nitrogen fixation, sulfidation, and other processes in the soil, promoting the decomposition of soil organic matter and the transformation of nutrients.

Biological nitrogen fixation is one of the most important ecological processes on Earth. In agricultural ecosystems, approximately 24% of the nitrogen in the total crop biomass is derived from the non-symbiotic nitrogen fixation process of the microorganisms. The rhizosphere is the most active area for microorganisms in agricultural soils, and the nitrogen-fixing microbial communities in the rhizosphere are closely related to the growth of crops. However, for a long time, the application of excessive chemical fertilizers and organic materials has greatly reduced the nitrogen fixation function of soil microorganisms in agricultural fields. In recent years, research on soil nitrogen-fixing microorganisms has mainly focused on nitrogen-fixing bacterial communities and their influencing factors. However, there has been little research on the mechanisms of how to regulate nitrogen fixation by rhizosphere microorganisms.

The nitrogen-fixing microorganisms refer to the microorganisms that can convert free nitrogen in the air into nitrogen-containing compounds. They are divided into free-living nitrogen-fixing bacteria and symbiotic nitrogen-fixing bacteria. The free-living nitrogen-fixing bacteria generally refer to strains that do not coexist with plants and have the function of nitrogen fixation, including *Actinomyces*, *Streptomyces*, *Micromonospora*, *Luteimonas* and so on; and the symbiotic nitrogen-fixing bacteria mainly include *Rhizobium*, *Mesorhizobium*, *Sinorhizobium*, *Frankia* and so on.

The nitrogen-fixing microorganisms play an important role in the growth of the crops, in particular in leguminous crops, providing a large amount of nitrogen nutrition for the growth of the crops. How to improve the abundance and nitrogen fixation capacity of nitrogen-fixing bacteria in the rhizosphere soil, increase the yields of the crops, and reduce fertilizer use has great development prospects.

### SUMMARY

The present disclosure aims to solve, at least to some extent, one of the technical problems in the related art. To this end, an object of the present disclosure is to provide a Streptomyces, which can increase the abundance of microorganisms with nitrogen-fixing function in the rhizosphere soil of the crops and increase the nitrogenase activity in the rhizosphere soil of the crops. The metabolites of this Streptomyces strain can be used to increase the abundance of nitrogen-fixing bacteria such as *Actinomyces, Streptomyces, Rhizobium, Mesorhizobium*, *Luteimonas*, and *Micromonospora* in the soil, increase the nitrogenase activity, increase the abundance of nitrogen-fixing microorganisms in the soil, increase the absorption of nitrogen nutrients by the crops, and improve the soil microecological environment. It has the potential to be developed into soil functional fertilizers or pesticides.

To this end, in a first aspect, the present disclosure provides a microorganism, which is *Streptomyces linyiensis* deposited on September 28, 2022 in China General Microbiological Culture Collection Center, CGMCC, under CGMCC accession No. 25834. The deposit address is Institute of Microbiology Chinese Academy of Sciences, No. 3, Yard 1, West Beichen Road, Chaoyang District, Beijing.

The Streptomyces provided in the present disclosure is a pure strain obtained from the soil of vegetable fields in Shandong Province by using a soil serial gradient dilution method combined with a plate streaking isolation method. Through field trials with tomato, cucumber, and cabbage, it was verified that the strain can effectively increase the abundance of nitrogen-fixing microorganisms in the rhizosphere soil of crops, for example, it can increase the abundance of nitrogen-fixing microorganisms such as *Actinomyces, Streptomyces, Rhizobium, Mesorhizobium,* and *Micromonospora.* The strain can be used in the preparation of a microbial agent, a composite microbial agent, a microbial fertilizer, or a biopesticide, which can increase the nitrogenase activity in the rhizosphere soil, improve the soil microecological environment, promote the growth of the crops, and increase the yield of the crops.

According to an embodiment of the present disclosure, the microorganism has the 16S rDNA sequence as set forth in SEQ ID NO: 1.

In a second aspect, the present disclosure provides a fermentation broth, and the fermentation broth is obtained by fermenting with the microorganism according to the first aspect.

In a third aspect, the present disclosure provides a microbial agent, and the microbial agent includes the microorganism according to the first aspect or the fermentation broth according to the second aspect of the present disclosure.

According to an embodiment of the present disclosure, the microbial agent is in the form of a dry powder, and a viable bacterial count of *Streptomyces linyiensis* in the microbial agent is at least 2×10⁹ CFU per gram of the microbial agent.

According to an embodiment of the present disclosure, the microbial agent is obtained by the following steps: fermenting and culturing the *Streptomyces linyiensis* to obtain a fermentation product; and performing spray-drying and pulverizing treatment on the fermentation product to obtain the microbial agent.

In a fourth aspect, the present disclosure provides a composite microbial agent, and the composite microbial agent includes the microbial agent according to the third aspect.

According to an embodiment of the present disclosure, the composite microbial agent includes a first microbial agent and a second microbial agent. The first microbial agent is the microbial agent according to the third aspect, and the second microbial agent includes at least one of *Bacillus subtilis*, *Bacillus amyloliquefaciens*, *Bacillus mucilaginosus*, *Streptomyces fradiae*, or *Streptomyces microflavus.*

According to an embodiment of the present disclosure, the composite microbial agent includes 10 to 50 parts by weight of the first microbial agent and 5 to 20 parts by weight of the second microbial agent. The second microbial agent includes 0 to 5 parts by weight of *Streptomyces microflavus,* 0 to 10 parts by weight of *Bacillus mucilaginosus,* 0 to 5 parts by weight of *Bacillus subtilis,* 0 to 5 parts by weight of *Bacillus amyloliquefaciens*, and 0 to 5 parts by weight of *Streptomyces fradiae.*

According to an embodiment of the present disclosure, in the composite microbial agent, a viable bacterial count of *Streptomyces linyiensis* is at least 2×10⁹ CFU per gram of the composite microbial agent, and at least one of the following conditions is satisfied: a viable bacterial count of *Streptomyces microflavus* is at least 5×10⁸ CFU per gram, a viable bacterial count of *Bacillus mucilaginosus* is at least 5×10⁸ CFU per gram, a viable bacterial count of *Bacillus subtilis* is at least 5×10⁸ CFU per gram, a viable bacterial count of *Bacillus amyloliquefaciens* is at least 5×10⁸ CFU per gram, or a viable bacterial count of *Streptomyces fradiae* is at least 5×10⁸ CFU per gram.

In a fifth aspect, the present disclosure provides a use of the microorganism according to the first aspect, the fermentation broth according to the second aspect, the microbial agent according to the third aspect, or the composite microbial agent according to the fourth aspect in the preparation of a microbial fertilizer or a biopesticide. The Streptomyces and the microbial agent and composite microbial agent containing the same according to the present disclosure can increase the abundance of nitrogen-fixing microorganisms in the rhizosphere soil of crops and increase the nitrogenase activity in the rhizosphere soil of the crops, and can be used in the preparation of the microbial fertilizer and/or the biopesticide to improve the soil microecological environment, promote the growth of the crops, and increase the yield of the crops.

In a sixth aspect, the present disclosure provides a microbial fertilizer. The microbial fertilizer includes the Streptomyces according to the first aspect, the fermentation broth according to the second aspect, the microbial agent according to the third aspect, or the composite microbial agent according to the fourth aspect.

According to an embodiment of the present disclosure, the microbial fertilizer is a solid fertilizer or a liquid fertilizer.

According to an embodiment of the present disclosure, the microbial fertilizer is a solid fertilizer, and a viable bacterial count in the microbial fertilizer is at least 2×10⁹ CFU per gram of the microbial fertilizer.

According to an embodiment of the present disclosure, the microbial fertilizer is a liquid fertilizer, and a viable bacterial count in the microbial fertilizer is at least 2×10⁹ CFU per liter of the microbial fertilizer.

According to an embodiment of the present disclosure, the microbial fertilizer further includes a base fertilizer, and the base fertilizer includes at least one of a compound fertilizer, a water-soluble fertilizer, an organic fertilizer, or a foliar fertilizer.

In a seventh aspect, the present disclosure provides a biopesticide, and the biopesticide includes the Streptomyces according to the first aspect, the fermentation broth according to the second aspect, the microbial agent according to the third aspect, or the composite microbial agent according to the fourth aspect.

According to an embodiment of the present disclosure, a viable bacterial count of the microorganisms contained in the biopesticide is at least 2×10⁹ CFU per liter.

In an eighth aspect, the present disclosure provides a use of the microorganism according to the first aspect, the fermentation broth according to the second aspect, the microbial agent according to the third aspect, the composite microbial agent according to the fourth aspect, the microbial fertilizer according to the sixth aspect, or the biopesticide according to the seventh aspect in the increase abundance of a nitrogen-fixing microorganism in rhizosphere soil of crops, or the increase of nitrogenase activity in the rhizosphere soil of the crops.

According to an embodiment of the present disclosure, the nitrogen-fixing microorganism includes at least one genus selected from *Actinomyces*, *Streptomyces*, *Rhizobium*, *Mesorhizobium*, *Micromonospora*, or *Luteimonas.*

In a ninth aspect, the present disclosure provides a method for increasing abundance of a nitrogen-fixing microorganism in rhizosphere soil of crops, which includes: applying the microorganism according to the first aspect, the fermentation broth according to the second aspect, the microbial agent according to the third aspect, the composite microbial agent according to the fourth aspect, the microbial fertilizer according to the sixth aspect, or the biopesticide according to the seventh aspect to the crops.

According to an embodiment of the present disclosure, the crops include at least one of corn, wheat, soybean, rice, potato, sorghum, cucumber, pepper, tomato, peanut, eggplant, or cotton.

In a tenth aspect, the present disclosure provides a method for increasing nitrogenase activity in rhizosphere soil of crops, which includes: applying the microorganism according to the first aspect, the fermentation broth according to the second aspect, the microbial agent according to the third aspect, the composite microbial agent according to the fourth aspect, the microbial fertilizer according to the sixth aspect, or the biopesticide according to the seventh aspect to the crops.

According to an embodiment of the present disclosure, the crops include at least one of corn, wheat, soybean, rice, potato, sorghum, cucumber, pepper, tomato, peanut, eggplant, or cotton.

The beneficial effects of the present disclosure relative to the prior art are as follows.

The Streptomyces provided in the present disclosure has the advantages of stable properties, simple cultivation, obvious effect, wide application conditions, and no environmental pollution. The Streptomyces can be used in the preparation of the microbial agent, the composite microbial agent, the microbial fertilizer, or the biopesticide to increase the abundance of nitrogen-fixing microorganisms in the rhizosphere soil of crops, increase the nitrogenase activity in the rhizosphere soil of the crops, improve the soil microecological environment, promote the growth of the crops, and increase the yield of the crops. It is particularly effective in crops such as tomatoes, cucumbers, and cabbages. The Streptomyces is beneficial to ensuring the planting and agricultural production of the crops and has great application value.

Additional aspects and advantages of the present disclosure will be provided in part in the following description, or will become apparent in part from the following description, or can be learned from practicing of the present disclosure.

### Deposit information:

Strain name: *Streptomyces linyiensis*
Date of deposit: September 28, 2022
Depository institution: China General Microbiological Culture Collection Center
Accession number: CGMCC No. 25834

### BRIEF DESCRIPTION OF THE DRAWINGS

The foregoing and/or additional aspects and advantages of the present disclosure will become apparent and readily appreciated from the following description of the embodiments in conjunction with the accompanying drawings, of which:
FIG. 1 shows a colony photo of *Streptomyces linyiensis* on a culture medium; and
FIG. 2 shows a schematic diagram of a phylogenetic tree of *Streptomyces linyiensis.*

### DETAILED DESCRIPTION

The embodiments of the present disclosure are described in detail below. The embodiments described below are exemplary and are intended to be illustrative of the present disclosure and are not to be construed as limiting the present disclosure.

Furthermore, the terms "first" and "second" are used for descriptive purposes only and cannot be construed as indicating or implying relative importance or implicitly indicating the number of technical features indicated. Thus, a feature defined as "first" or "second" may explicitly or implicitly include at least one such feature. In the description of the present disclosure, the meaning of "plurality" means at least two, e.g. two, three, etc. unless clearly and specifically defined otherwise.

According to an embodiment of the present disclosure, a first aspect of the present disclosure provides a microorganism, which is *Streptomyces linyiensis* deposited on September 28, 2022 in China General Microbiological Culture Collection Center, under CGMCC accession No. 25834. The strain was screened from the soil of vegetable fields in Shandong Province. Based on colony morphology observation, cell Gram staining, physiological and biochemical characteristics detection, and 16S rDNA gene analysis, the strain was identified as a new species. According to the international naming rules, the strain was named *Streptomyces linyiensis.* The Streptomyces according to the present disclosure can effectively increase the abundance of nitrogen-fixing microorganisms in the rhizosphere soil of crops, for example, it can increase the abundance of *Actinomyces*, *Streptomyces*, *Rhizobium*, *Mesorhizobium*, *Luteimonas,* and *Micromonospora.* The strain can be used in the preparation of a microbial agent, a composite microbial agent, a microbial fertilizer, or a biopesticide to increase the abundance of nitrogen-fixing microorganisms in the rhizosphere soil of the crops, increase the nitrogenase activity in the rhizosphere soil of the crops, improve the soil microecological environment, promote the growth of the crops, and increase the yield of the crops.

According to an embodiment of the present disclosure, the microorganism has the 16S rDNA sequence as set forth in SEQ ID NO: 1.

According to an embodiment of the present disclosure, a second aspect of the present disclosure provides a fermentation broth, which is obtained by fermenting with the microorganism according to the first aspect.

According to an embodiment of the present disclosure, a third aspect of the present disclosure provides a microbial agent, which includes the microorganism according to the first aspect or the fermentation broth according to the second aspect of the present disclosure.

According to an embodiment of the present disclosure, the microbial agent is in the form of a dry powder, and viable bacterial count of *Streptomyces linyiensis* in the microbial agent is at least 2×10⁹ CFU per gram of the microbial agent.

According to an embodiment of the present disclosure, the microbial agent is obtained by the following steps: fermenting and culturing the *Streptomyces linyiensis* to obtain a fermentation product; and performing spray-drying and pulverizing treatment on the fermentation product to obtain the microbial agent.

According to an embodiment of the present disclosure, a fourth aspect of the present disclosure provides a composite microbial agent, which includes the microbial agent according to the third aspect.

According to an embodiment of the present disclosure, the composite microbial agent includes a first microbial agent and a second microbial agent. The first microbial agent is the microbial agent according to the third aspect, and the second microbial agent includes at least one of *Bacillus subtilis*, *Bacillus amyloliquefaciens*, *Bacillus mucilaginosus*, *Streptomyces fradiae*, or *Streptomyces microflavus.*

According to an embodiment of the present disclosure, the composite microbial agent includes 10 to 50 parts by weight of the first microbial agent and 5 to 20 parts by weight of the second microbial agent. The second microbial agent includes 0 to 5 parts by weight of *Streptomyces microflavus,* 0 to 10 parts by weight of *Bacillus mucilaginosus,* 0 to 5 parts by weight of *Bacillus subtilis,* 0 to 5 parts by weight of *Bacillus amyloliquefaciens*, and 0 to 5 parts by weight of *Streptomyces fradiae.*

According to an embodiment of the present disclosure, in the composite microbial agent, a viable bacterial count of *Streptomyces linyiensis* is at least 2×10⁹ CFU per gram of the composite microbial agent, and at least one of the following conditions is satisfied: a viable bacterial count of *Streptomyces microflavus* is at least 5×10⁸ CFU per gram, a viable bacterial count of *Bacillus mucilaginosus* is at least 5×10⁸ CFU, a viable bacterial count of *Bacillus subtilis* is at least 5×10⁸ CFU per gram, a viable bacterial count of *Bacillus amyloliquefaciens* is at least 5×10⁸ CFU per gram, or a viable bacterial count of *Streptomyces fradiae* is at least 5×10⁸ CFU per gram.

According to an embodiment of the present disclosure, a fifth aspect of the present disclosure provides a use of the microorganism according to the first aspect, the fermentation broth according to the second aspect, the microbial agent according to the third aspect, or the composite microbial agent according to the fourth aspect in the preparation of a microbial fertilizer or a biopesticide.

According to an embodiment of the present disclosure, a sixth aspect of the present disclosure provides a microbial fertilizer, which includes the microorganism according to the first aspect, the fermentation broth according to the second aspect, the microbial agent according to the third aspect, or the composite microbial agent according to the fourth aspect.

According to an embodiment of the present disclosure, the microbial fertilizer is a solid fertilizer or a liquid fertilizer.

According to an embodiment of the present disclosure, the microbial fertilizer is a solid fertilizer, and a viable bacterial count in the microbial fertilizer is at least 2×10⁹ CFU per gram.

According to an embodiment of the present disclosure, the microbial fertilizer is a liquid fertilizer, and a viable bacterial count in the microbial fertilizer is at least 2×10⁹ CFU per liter.

According to an embodiment of the present disclosure, the microbial fertilizer further includes a base fertilizer, and the base fertilizer includes at least one of a compound fertilizer, a water-soluble fertilizer, an organic fertilizer, or a foliar fertilizer.

According to an embodiment of the present disclosure, a seventh aspect of the present disclosure provides a biopesticide, which includes the Streptomyces according to the first aspect, the fermentation broth according to the second aspect, the microbial agent according to the third aspect, or the composite microbial agent according to the fourth aspect.

According to an embodiment of the present disclosure, a viable bacterial count in the biopesticide is at least 2×10⁹ CFU per liter.

According to an embodiment of the present disclosure, an eighth aspect of the present disclosure provides a use of the microorganism according to the first aspect, the fermentation broth according to the second aspect, the microbial agent according to the third aspect, the composite microbial agent according to the fourth aspect, the microbial fertilizer according to the sixth aspect, or the biopesticide according to the seventh aspect in the increase of abundance of a nitrogen-fixing microorganism in rhizosphere soil of crops, or the increase of nitrogenase activity in the rhizosphere soil of the crops.

According to an embodiment of the present disclosure, the nitrogen-fixing microorganism includes at least one genus of *Actinomyces, Streptomyces, Rhizobium, Mesorhizobium*, *Micromonospora*, or *Luteimonas.*

According to an embodiment of the present disclosure, a ninth aspect of the present disclosure provides a method for increasing abundance of a nitrogen-fixing microorganism in rhizosphere soil of crops, which includes: applying the microorganism according to the first aspect, the fermentation broth according to the second aspect, the microbial agent according to the third aspect, the composite microbial agent according to the fourth aspect, the microbial fertilizer according to the sixth aspect, or the biopesticide according to the seventh aspect to the crops.

According to an embodiment of the present disclosure, the crops include at least one selected from corn, wheat, soybean, rice, potato, sorghum, cucumber, pepper, tomato, peanut, eggplant, or cotton.

According to an embodiment of the present disclosure, a tenth aspect of the present disclosure provides a method for increasing nitrogenase activity in rhizosphere soil of crops, which includes: applying the microorganism according to the first aspect, the fermentation broth according to the second aspect, the microbial agent according to the third aspect, the composite microbial agent according to the fourth aspect, the microbial fertilizer according to the sixth aspect, or the biopesticide according to the seventh aspect to the crops.

According to an embodiment of the present disclosure, the crops include at least one of corn, wheat, soybean, rice, potato, sorghum, cucumber, pepper, tomato, peanut, eggplant, or cotton.

Where specific techniques or conditions are not specified in the examples, they are performed according to techniques or conditions described in the literature in the art or according to the product description. The reagents or instruments used without indicating the manufacturer are conventional products that can be obtained commercially.

### Example 1. Isolation and purification of Streptomyces

### (1) Strain isolation

Soil was collected from vegetable fields in a city of Shandong Province and placed in sterile sample bags and stored at 4°C for later use. The strains were isolated using the soil dilution coating plate method. The collected soil was mixed evenly. 10 g of the soil was taken, added to sterile water, and made up the volume to 100 ml. The mixture was shaken on a shaker for 30 min, and the shaker parameters were set to 30°C and 180 rpm. After shaking evenly, 1 ml of soil suspension was aspirated and added to 9 ml of sterile water to prepare a soil suspension with a concentration of 10⁻². After mixing evenly, 1 ml of the soil suspension with the concentration of 10⁻² was taken using a sterile pipette and added to 9 ml of sterile water. This process was repeated to prepare dilutions with concentrations of 10⁻³, 10⁻⁴, 10⁻⁵, and 10⁻⁶. 0.1 ml of soil dilution at each concentration gradient was aspirated and added to Gao's synthetic agar medium No. 1, and the dilution was spread evenly on the surface of the medium using a spreader, with 3 replicates for each concentration, and incubated at a constant temperature of 30°C.

### (2) Strain purification

After culturing on the coated plate for 5 to 7 days, a single colony was picked with an inoculation loop, purified and cultured on Gao's medium No. 1 by using the plate streak isolation method, and cultured at a constant temperature of 30°C for 5 to 15 days. The same method was used for purification twice again to obtain pure strain F9.

### Example 2. Identification of Streptomyces

The pure strain obtained was identified by morphology, physiology, biochemistry, and molecular biology, respectively. The results were as follows:

### (1) Morphological characteristics:

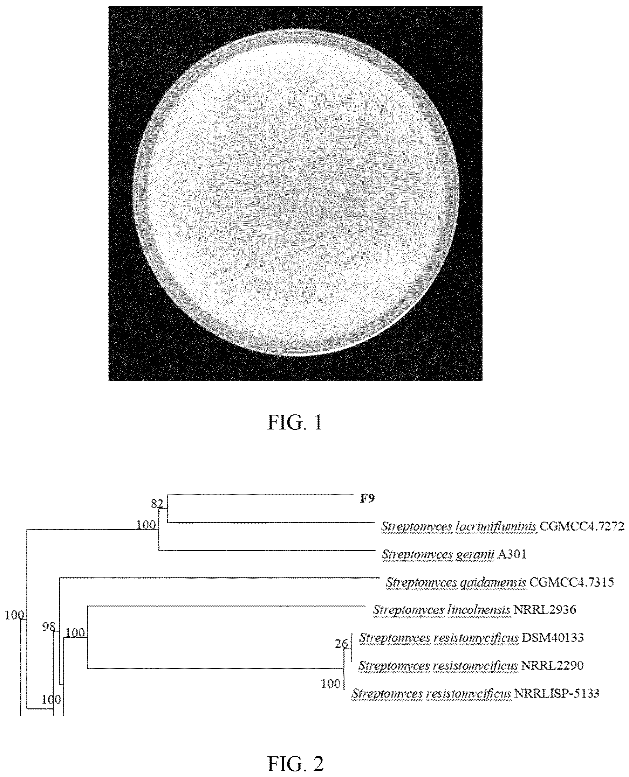

The colonies were dry and opaque, with white irregular protrusions on the surface and blue spores. They were tightly attached to the culture medium and were not easy to picked up, and werere not easy to break after being picked up. The colors of the front and back of the colonies are inconsistent; and the plane of the culture medium at the edge of the colonies was deformed. The outer wall was smooth and uniform in texture, and the bacterial cells were filamentous with multiple branches, as shown in FIG. 1.

### (2) Physiological and biochemical characteristics:

The fatty acid composition of the strain was detected by microbial fatty acid rapid identification system (MIDI). The results showed that the main fatty acids of the strain were C14:0 iso, C16:0, C16:0 iso, C15:0 anteiso, and C15:0 iso, with contents of 16.21%, 15.35%, 14.94%, 11.12%, and 6.11%, respectively. The specific fatty acid composition results were shown in Table 1, and the results indicated that the purified strain screened in the present disclosure conformed to the main cellular fatty acid characteristics of the Streptomyces.

**[Table 1] Streptomyces fatty acid data**

| Name | Content | Comment 1 | Comment 2 |
|---|---|---|---|
| 10:0 iso | 0.67 | - | - |
| 10:0 | 0.35 | - | - |
| 12:0 iso | 1.15 | - | - |
| 12:0 | 1.14 | - | - |
| 13:0 iso | 1.78 | - | - |
| 13:0 anteiso | 1.81 | - | - |
| 13:0 | 0.39 | - | - |
| 14:0 iso | 16.21 | - | - |
| 14:0 anteiso | 0.28 | - | - |
| 14:1 ω5c | 0.27 | - | - |
| 14:0 | 3.27 | - | - |
| 15:0 iso | 6.11 | - | - |
| 15:0 anteiso | 11.12 | - | - |
| 15:1 ω6c | 0.41 | - | - |
| 16:1 iso H | 2.48 | - | - |
| 16:0 iso | 14.94 | - | - |
| 16:0 | 15.35 | - | - |
| 15:0 iso 3OH | 0.69 | - | - |
| 15:0 2OH | 0.25 | - | - |
| 17:1 anteiso ω9c | 1.44 | - | - |
| 17:0 iso | 1.57 | - | - |
| 17:0 anteiso | 2.73 | - | - |
| 17:1 ω8c | 0.55 | - | - |
| 17:0 cyclo | 1.50 | - | - |
| 17:0 | 0.88 | - | - |
| 16:0 3OH | 0.38 | - | - |
| 18:0 iso | 0.42 | - | - |
| 18:0 | 0.52 | - | - |
| 19:0 iso | 0.72 | - | - |
| 18:1 2OH | 0.27 | - | - |
| Combined characteristic 3 | 7.90 | 16:1 ω7c/16:1 ω6c | 16:1 ω6c/16:1 ω7c |
| Combined characteristic 5 | 0.69 | 18:0 ante/18:2 ω6,9c | 18:2 ω6,9c/18:0 ante |
| Combined characteristic 8 | 0.27 | 18:1 ω7c | 18:1 ω6c |
| Combined characteristic 9 | 1.52 | 16:0 10-methyl | 17:1 iso ω9c |

The results of Biolog GENIII test were shown in Table 2, which showed that the purified strain conformed to the biochemical metabolic characteristics of Streptomyces.

**[Table 2] Streptomyces Biolog GENIII data**

| Physiological and biochemical indicators (Biolog) | | Result |
|---|---|---|
| Negative Control | Negative Control | - |
| Dextrin | Dextrin | + |
| D-Maltose | D-Maltose | - |
| D-Trehalose | D-Trehalose | - |
| D-Cellobiose | D-Cellobiose | + |
| Gentiobiose | Gentiobiose | - |
| Sucrose | Sucrose | + |
| D-Turanose | D-Turanose | + |
| Stachyose | Stachyose | - |
| Positive Control | Positive Control | + |
| pH 6 | pH 6 | + |
| pH 5 | pH 5 | - |
| D-Raffinose | D-Raffinose | + |
| α-D-Lactose | α-D-Lactose | + |
| D-Melibiose | D-Melibiose | - |
| β-Methyl-DGlucoside | β-Methyl-DGlucoside | - |
| D-Salicin | D-Salicin | W |
| N-Acetyl-DGlucosamine | N-Acetyl-DGlucosamine | + |
| N-Acetyl-β-DMannosamine | N-Acetyl-β-DMannosamine | - |
| N-Acetyl-DGalactosamine | N-Acetyl-DGalactosamine | - |
| N-Acetyl Neuraminic Acid | N-Acetyl Neuraminic Acid | - |
| 1% NaCl | 1% NaCl | + |
| 4% NaCl | 4% NaCl | - |
| 8% NaCl | 8% NaCl | - |
| α-D-Glucose | α-D-Glucose | + |
| D-Mannose | D-Mannose | + |
| D-Fructose | D-Fructose | + |
| D-Galactose | D-Galactose | - |
| 3-Methyl Glucose | 3-Methyl Glucose | - |
| D-Fucose | D-Fucose | W |
| L-Fucose | L-Fucose | - |
| L-Rhamnose | L-Rhamnose | - |
| Inosine | Inosine | - |
| 1% Sodium Lactate | 1% Sodium Lactate | - |
| Fusidic Acid | Fusidic Acid | - |
| D-Serine | D-Serine | - |
| D-Sorbitol | D-Sorbitol | + |
| D-Mannitol | D-Mannitol | + |
| D-Arabitol | D-Arabitol | - |
| myo-Inositol | myo-Inositol | + |
| Glycerol | Glycerol | + |
| D-Glucose-6-PO4 | D-Glucose-6-PO4 | - |
| D-Fructose-6-PO4 | D-Fructose-6-P04 | + |
| D-Aspartic Acid | D-Aspartic Acid | - |
| D-Serine | D-Serine | - |
| Troleandomycin | Troleandomycin | - |
| Rifamycin SV | Rifamycin SV | - |
| Minocycline | Minocycline | - |
| Gelatin | Gelatin | + |
| Glycyl-L-Proline | Glycyl-L-Proline | W |
| L-Alanine | L-Alanine | W |
| L-Arginine | L-Arginine | + |
| L-Aspartic Acid | L-Aspartic Acid | - |
| L-Glutamic Acid | L-Glutamic Acid | W |
| L-Histidine | L-Histidine | - |
| L-Pyroglutamic Acid | L-Pyroglutamic Acid | - |
| L-Serine | L-Serine | - |
| Lincomycin | Lincomycin | - |
| Guanidine HCl | Guanidine HCl | - |
| Niaproof 4 | Niaproof 4 | - |
| Pectin | Pectin | + |
| D-Galacturonic Acid | D-Galacturonic Acid | W |
| L-Galactonic Acid Lactone | L-Galactonic Acid Lactone | - |
| D-Gluconic Acid | D-Gluconic Acid | + |
| D-Glucuronic Acid | D-Glucuronic Acid | + |
| Glucuronamide | Glucuronamide | W |
| Mucic Acid | Mucic Acid | - |
| Quinic Acid | Quinic Acid | - |
| D-Saccharic Acid | D-Saccharic Acid | - |
| Vancomycin | Vancomycin | - |
| Tetrazolium Violet | Tetrazolium Violet | - |
| Tetrazolium Blue | Tetrazolium Blue | - |
| p-Hydroxy-Phenylacetic Acid | p-Hydroxy-Phenylacetic Acid | - |
| Methyl Pyruvate | Methyl Pyruvate | - |
| D-Lactic Acid Methyl Ester | D-Lactic Acid Methyl Ester | W |
| L-Lactic Acid | L-Lactic Acid | - |
| Citric Acid | Citric Acid | - |
| α-Keto-Glutaric Acid | α-Keto-Glutaric Acid | + |
| D-Malic Acid | D-Malic Acid | - |
| L-Malic Acid | L-Malic Acid | - |
| Bromo-Succinic Acid | Bromo-Succinic Acid | - |
| Nalidixic Acid | Nalidixic Acid | + |
| Lithium Chloride | Lithium Chloride | - |
| Potassium Tellurite | Potassium Tellurite | + |
| Tween 40 | Tween 40 | + |
| γ-Amino-Butryric Acid | γ-Amino-Butryric Acid | + |
| α-Hydroxy-Butyric Acid | α-Hydroxy-Butyric Acid | + |
| β-Hydroxy-D,L-butyric Acid | β-Hydroxy-D,L-butyric Acid | + |
| α-Keto-Butyric Acid | α-Keto-Butyric Acid | + |
| Acetoacetic Acid | Acetoacetic Acid | - |
| Propionic Acid | Propionic Acid | - |
| Acetic Acid | Acetic Acid | - |
| Formic Acid | Formic Acid | - |
| Aztreonam | Aztreonam | + |
| Sodium Butyrate | Sodium Butyrate | - |
| Sodium Bromate | Sodium Bromate | - |

| | | |
|---|---|---|
| Note: + indicates positive; - indicates negative; w indicates weakly positive. | | |

### (3) Molecular biological characteristics:

1) The basic characteristics of the genome of the purified strain were analyzed as follows.

The genome size of strain F9 obtained was 11,175,236 bp, and the G+C content was 70.21%. The genome characteristics were basically consistent with other species of the genus Streptomyces.

**[Table 3] Analysis of basic characteristics of purified strain genome**

| Characteristic | Total |
|---|---|
| Scaffold number | 198 |
| Total length (bp) | 11,175,236 |
| GC % | 70.21 |
| *N50* (bp) | 116,251 |
| *N75* (bp) | 71,817 |
| sRNA | 51 |
| tRNA | 66 |
| rRNA | 18 |

2) The 16S rDNA gene sequence (1416 bp) and the analysis results of phylogenetic tree of the purified strain were as follows.

The 16S rDNA gene sequence of the purified strain was determined by using universal primers 27F and 1492R (27F 5'-GTTTGATCMTGGCTCAG-3', SEQ ID NO: 2; 1492R 5'-TACGGYTACCTTGTTACGACTT-3', SEQ ID NO: 3) of bacterial 16S rDNA gene to obtain a gene fragment of 1416 bp:

TCGAACGATGAAGCCTTTCGGGGTGGATTAGTGGCGAACGGGTGAGTA
ACACGTGGGCAATCTGCCCTGCACTCTGGGACAAGCCCTGGAAACGGGGTCTAAT
ACCGGATGATACCTGCCGAGGCATCTCGGTGGGTTGAAAGCTCCGGCGGTGCAGG
ATGAGCCCGCGGCCTATCAGCTTGTTGGTGAGGTAGTGGCTCACCAAGGCGACGA
CGGGTAGCCGGCCTGAGAGGGCGACCGGCCACACTGGGACTGAGACACGGCCCA
GACTCCTACGGGAGGCAGCAGTGGGGAATATTGCACAATGGGCGAAAGCCTGATG
CAGCGACGCCGCGTGAGGGATGACGGCCTTCGGGTTGTAAACCTCTTTCAGCAGG
GAAGAAGCGCAAGTGACGGTACCTGCAGAAGAAGCGCCGGCTAACTACGTGCCA

GCAGCCGCGGTAATACGTAGGGCGCAAGCGTTGTCCGGAATTATTGGGCGTAAAG

AGCTCGTAGGCGGTCTGTCGCGTCGGATGTGAAAGCCCGGGGCTTAACCCCGGGT

CTGCATTCGATACGGGCAGACTAGAGTGTGGTAGGGGAGATCGGAATTCCTGGTGT

AGCGGTGAAATGCGCAGATATCAGGAGGAACACCGGTGGCGAAGGCGGATCTCTG

GGCCATTACTGACGCTGAGGAGCGAAAGCGTGGGGAGCGAACAGGATTAGATACC

CTGGTAGTCCACGCCGTAAACGGTGGGAACTAGGTGTTGGCGACATTCCACGTCG

TCGGTGCCGCAGCTAACGCATTAAGTTCCCCGCCTGGGGAGTACGGCCGCAAGGC

TAAAACTCAAATGGAATTGACGGGGGCCCGCACAAGCAGCGGAGCATGTGGCTTA

ATTCGACGCAACGCGAAGAACCTTACCAAGGCTTGACATACACCGGAAACGGCCA

GΛGΛTGGTCGCΛCCCCTTGTGGTCGGTGTΛCΛGGTGGTGCΛTGGCTGTCGTCΛGCT

CGTGTCGTGAGATGTTGGGTTAAGTCCCGCAACGAGCGCAACCCTTGTTCTGTGTT

GCCAGCATGCCCTTCGGGGTGATGGGGACTCACAGGAGACTGCCGGGGTCAACT

CGGAGGAAGGTGGGGACGACGTCAAGTCATCATGCCCCTTATTGTCTTGGGCTGC

ACACGTGCTACAATGGCCGGTACAATGAGCTGCGATGCCGTGAGGCGGAGCGAAT

CTCAAAAAGCCGGTCTCAGTTCGGATTGGGGTCTGCAACTCGACCCCATGAAGTC

GGAGTTGCTAGTAATCGCAGATCAGCATTGCTGCGGTGAATACGTTCCCGGGCCTT

GTACACACCGCGTCACGAAAGTCGGTAACACCCGAAGCCGGTGGCCCAACCCCTG

TAAGGGAGGGAGCTGTCGAAAGGTGGGACTGGCGATTGGGACGAAG (SEQ ID NO:

1)

The gene sequence thereof was compared with those of known type strains, and the results were shown in Table 4. The purified strain had the highest similarity with the known *Streptomyces geranii* type species A301T, which was 99.34%.

**[Table 4] Comparative analysis of 16S rRNA gene sequences of purified strain**

| Serial number | Name | Strain | Accession | Pairwise similarity (%) |
|---|---|---|---|---|
| 1 | *Streptomyces geranii* | A301 | MF100124 | 99.34 |
| 2 | *Streptomyces turgidiscabies* | ATCC 700248 | AB026221 | 98.83 |
| 3 | *Streptomyces reticuliscabiei* | NRRL B-24446 | MUNI0100032 5 | 98.83 |
| 4 | *Streptomyces cinereoruber subsp. fructofermentans* | NBRC 15396 | AB184647 | 98.60 |
| 5 | *Streptomyces mirabilis* | NBRC 13450 | AB184412 | 98.32 |
| 6 | *Streptomyces eurocidicus* | NRRL B-1676 | AY999790 | 98.32 |
| 7 | *Streptomyces europaeiscabiei* | KACC 20186 | AY207598 | 98.25 |
| 8 | *Streptomyces zhihengii* | YIM T102 | KU936048 | 98.25 |
| 9 | *Streptomyces kaempferi* | 137 | HE591382 | 98.25 |
| 10 | *Streptomyces scabiei* | NRRL B-16523 | LBNJ01000196 | 98.17 |
| 11 | *Streptomyces lacrimifluminis* | Z1027 | KJ829342 | 98.17 |
| 12 | *Streptomyces phaeolivaceus* | GY16 | MT555308 | 98.17 |
| 13 | *Streptomyces polymachus* | T258 | KM229363 | 98.17 |
| 14 | *Streptomyces rishiriensis* | NBRC 13407 | AB184383 | 98.10 |
| 15 | *Streptomyces blastmyceticus* | NRRL B-5480 | AY999802 | 98.10 |

The phylogenetic tree constructed from its 16S rDNA gene sequence and related groups was shown in FIG. 2. The results showed that the strain was clustered into the same branch as *Streptomyces geranii* A301^{T}.

3) The ANI value of the purified strain was analyzed using the JSpeciesWS website. The results were shown in Table 5. It can be concluded that the ANI of this strain and the type strain A301^{T} of the known species *Streptomyces geranii* was 87.29%. Moreover, the ANI values of the strain and its related species were all lower than 95%. Generally speaking, the ANI value between strains of the same species is ≥95%. Therefore, it can be concluded that the purified strain should be a new species. According to the international naming rules, the strain was named *Streptomyces linyiensis.*

**[Table 5] Analysis of Streptomyces ANI values**

| | Strain | *Streptomyces geranii* A301^{T} | *Streptomyces eurocidicus* ATCC 27428^{T} | *Streptomyces mirabilis* WAC00263^{T} | *Streptomyces reticuliscabiei* NRRL B-24446^{T} |
|---|---|---|---|---|---|
| Strain | 100 | 87.29 | 74.82 | 80.73 | 87.14 |
| *Streptomyces gerani*i A301 ^{T} | | 100 | 74.63 | 80.53 | 86.47 |
| *Streptomyces eurocidicus* ATCC 27428^{T} | | | 100 | 76.93 | 75.72 |
| *Streptomyces mirabilis* WAC00263^{T} | | | | 100 | 81.27 |
| *Streptomyces reticuliscabiei* NRRL B-24446^{T} | | | | | 100 |
| *Streptomyces lacrimifluminis* CGMCC 4.7272^{T} | | | | | |

### Example 3. Effects of Streptomyces linyiensis fermentation broth on tomato yield and rhizosphere microbial community structure

First, the *Streptomyces linyiensis* fermentation broth was prepared according to the following method.

The Streptomyces was fermented in Gao's No. 1 liquid culture medium at a temperature of 30°C and a rotation speed of 180 rpm for 4 days to obtain the Streptomyces fermentation broth.

A batch of tomato field experiments were arranged and divided into two groups, namely a control group and a treatment group. The control group was treated by flushing with clean water, and the treatment group was treated by flushing with Streptomyces liquid fermentation broth. 90 days after planting, tomatoes were harvested, rhizosphere soil was collected, soil metagenome was measured, and the rhizosphere microbial community structure was analyzed. The results showed that compared with the control group, the tomato yield was increased by 16.7%, the rhizosphere nitrogenase activity was increased by 20%, and the nitrogen absorption by the plant was increased by 13% in the treatment group. The rhizosphere microbial community structure was analyzed, and the results were shown in Table 6. Compared with the control group, the abundance of *Actinomyces* in the rhizosphere soil was increased by 8.6%, the abundance of *Streptomyces* was increased by 9.1%, the abundance of *Rhizobium* was increased by 12.8%, the abundance of *Mesorhizobium* was increased by 6.7%, and the abundance of *Micromonospora* was increased by 14.1% in the treatment group.

**[Table 6] Effects of Streptomyces fermentation broth on tomato yield and rhizosphere microbial community structure**

| | Original soil | Control group | Streptomyces treatment group |
|---|---|---|---|
| Yield (Kg) | - | 2.46 | 2.87 |
| Nitrogenase activity (U/g) | - | 361.14 | 433.54 |
| Nitrogen absorption (g) | - | 97.7 | 110.7 |
| Abundance of *Actinomyces* | 1.21% | 1.28% | 1.39% |
| Abundance of *Streptomyces* | 1.25% | 1.32% | 1.44% |
| Abundance of *Rhizobium* | 0.85% | 0.86% | 0.97% |
| Abundance of *Mesorhizobium* | 0.87% | 0.89% | 0.95% |
| Abundance of *Micromonospora* | 0.84% | 0.85% | 0.97% |

### Example 4. Effects of a microbial agent containing Streptomyces on corn yield and rhizosphere microbial community structure

First, the microbial agent was prepared according to the following method.

The Gao's No. 1 liquid culture medium was added to the bran with a solid-liquid mass ratio of 4:1 to prepare a semi-solid culture medium. After inoculating Streptomyces, the culture medium was fermented at 30°C for 5 days, dried at 40°C, and crushed to obtain a Streptomyces agent. The viable bacterial count of *Streptomyces linyiensis* in the microbial agent was 2×10⁹ CFU.

A batch of corn field experiments were arranged and divided into two groups, namely a control group and a treatment group. The control group was treated by flushing with clean water, and the treatment group was treated by flushing with the microbial agent containing Streptomyces. 90 days after planting, the yield was measured, rhizosphere soil was collected, soil metagenome was measured, and the rhizosphere microbial community structure was analyzed. The results showed that compared with the control group, the corn yield was increased by 5.3%, the rhizosphere nitrogenase activity was increased by 24.3%, and nitrogen absorption by the plant was increased by 8.0% in the treatment group. The rhizosphere microbial community structure was analyzed, and the results were shown in Table 7. Compared with the control group, the abundance of *Actinomyces* was increased by 4.3%, the abundance of *Streptomyces* was increased by 9.9%, the abundance of *Rhizobium* was increased by 16%, the abundance of *Mesorhizobium was* increased by 7.9%, and the abundance of *Micromonospora* was increased by 10.3% in the rhizosphere soil of the treatment group.

**[Table 7] Effects of Streptomyces fermentation broth on corn yield and rhizosphere microbial community structure**

| | Original soil | Control group | Streptomyces treatment group |
|---|---|---|---|
| Yield (Kg) | - | 254.03 | 267.46 |
| Nitrogenase activity (U/g) | - | 313.25 | 389.37 |
| Nitrogen absorption (Kg) | - | 10.16 | 10.97 |
| Abundance of *Actinomyces* | 1.15% | 1.17% | 1.22% |
| Abundance of *Streptomyces* | 1.02% | 1.01% | 1.11% |
| Abundance of *Rhizobium* | 0.73% | 0.75% | 0.87% |
| Abundance of *Mesorhizobium* | 0.87% | 0.89% | 0.96% |
| Abundance of *Micromonospora* | 1.06% | 1.07% | 1.18% |

### Example 5. Effect of a liquid fertilizer containing Streptomyces on cabbage yield and rhizosphere microbial community structure

The liquid microbial fertilizer was prepared by mixing the Streptomyces fermentation broth with nutrients such as potassium dihydrogen phosphate, urea, monoammonium phosphate, potassium sulfate, potassium humate, and trace elements. The liquid microbial fertilizer was applied to cabbage plants to verify the effect. A batch of cabbage field experiments were arranged and divided into two groups, namely a control group and a treatment group. The control group was fertilized with isonutrient fertilizers, and the treatment group was fertilized with isonutrient microbial fertilizers containing Streptomyces fermentation broth. After 60 days of growth, the yield was measured, rhizosphere soil was collected, soil metagenome was measured, and the rhizosphere microbial community structure was analyzed. The results showed that compared with the control group, the cabbage yield was increased by 19.7%, the rhizosphere nitrogenase activity was increased by 23.1%, and nitrogen absorption by the plant was increased by 15.5% in the treatment group. The rhizosphere microbial community structure was analyzed, and the results were shown in Table 8. Compared with the control group, the abundance of *Actinomyces* was increased by 10.5%, the abundance of *Streptomyces* was increased by 16.7%, the abundance of *Rhizobium* was increased by 11.9%, the abundance of *Mesorhizobium* was increased by 12.6%, and the abundance of *Luteimonas* was increased by 9.6% in the rhizosphere soil of the treatment group.

**[Table 8] Effects of liquid microbial fertilizer containing Streptomyces on cabbage yield and rhizosphere microbial community structure**

| | Original soil | Control group | Streptomyces treatment group |
|---|---|---|---|
| Yield (Kg) | - | 120.45 | 144.23 |
| Nitrogenase activity (U/g) | - | 284.12 | 349.77 |
| Nitrogen absorption (Kg) | - | 3.61 | 4.17 |
| Abundance of *Actinomyces* | 1.43% | 1.52% | 1.68% |
| Abundance of *Streptomyces* | 1.08% | 1.08% | 1.26% |
| Abundance of *Rhizobium* | 0.56% | 0.59% | 0.66% |
| Abundance of *Mesorhizobium* | 0.94% | 0.95% | 1.07% |
| Abundance of *Luteimonas* | 1.13% | 1.14% | 1.25% |

### Example 6. Effect of solid fertilizer containing Streptomyces on cabbage rhizosphere microbial community structure

The solid microbial fertilizer was prepared by mixing Streptomyces solid microbial agent with nutrients such as potassium dihydrogen phosphate, urea, monoammonium phosphate, potassium sulfate, potassium humate, and trace elements. The solid microbial fertilizer was applied to cabbage plants to verify the effect. A batch of cabbage field experiments were arranged and divided into two groups, namely a control group and a treatment group. The control group was fertilized with isonutrient fertilizers, and the treatment group was fertilized with isonutrient microbial fertilizers containing Streptomyces fermentation broth. After 60 days of growth, the yield was measured, rhizosphere soil was collected, soil metagenome was measured, and the rhizosphere microbial community structure was analyzed. The results showed that compared with the control group, the cabbage yield was increased by 19.8%, the rhizosphere nitrogenase activity was increased by 24.1%, and the nitrogen absorption by the plant was increased by 16.4% in the treatment group. The rhizosphere microbial community structure was analyzed, and the results were shown in Table 9. Compared with the control group, the abundance of *Actinomyces* was increased by 9.6%, the abundance of *Streptomyces* was increased by 9.6%, the abundance of *Rhizobium* was increased by 10.4%, the abundance of *Mesorhizobium* was increased by 13.2%, and the abundance of *Luteimonas* was increased by 10.5% in the rhizosphere soil of the treatment group.

**[Table 9] Effects of solid microbial fertilizer containing Streptomyces on cabbage yield and rhizosphere microbial community structure**

| | Original soil | Control group | Streptomyces treatment group |
|---|---|---|---|
| Yield (Kg) | - | 165.32 | 198.08 |
| Nitrogenase activity (U/g) | - | 284.12 | 352.51 |
| Nitrogen absorption (Kg) | - | 4.95 | 5.76 |
| Abundance of *Actinomyces* | 1.24% | 1.26% | 1.38% |
| Abundance of *Streptomyces* | 1.03% | 1.04% | 1.14% |
| Abundance of *Rhizobium* | 0.76% | 0.77% | 0.85% |
| Abundance of *Mesorhizobium* | 0.89% | 0.91% | 1.03% |
| Abundance of *Luteimonas* | 0.93% | 0.95% | 1.05% |

### Example 7. Effect of a composite microbial agent containing Streptomyces on cucumber yield and rhizosphere microbial community structure

First, the microbial agent was prepared according to the following method.

The Gao's No. 1 liquid culture medium was added to the bran with a solid-liquid mass ratio of 4:1 to prepare a semi-solid culture medium. After inoculating Streptomyces, the culture medium was fermented at 30°C for 5 days, dried at 40°C, and crushed to obtain a Streptomyces powder. The Streptomyces powder was compounded with *Streptomyces microflavus* and *Bacillus mucilaginosus* powder to obtain the composite microbial agent 1. The *Streptomyces* powder was compounded with *Streptomyces microflavus* powder, *Bacillus mucilaginosus* powder, *Bacillus subtilis* powder, *Bacillus amyloliquefaciens* powder, and *Streptomyces fradiae* powder to obtain composite microbial agent 2. The viable bacterial count of *Streptomyces linyiensis* in the composite microbial agents 1 and 2 were both 2×10⁹ CFU.

A batch of cucumber field experiments were arranged and divided into two groups, namely a control group and a treatment group. The control group was treated by flushing with clean water, and the treatment group was treated by flushing with a composite microbial agent containing Streptomyces. After 90 days of planting, the yield was measured, the rhizosphere soil was collected, the soil metagenome was measured, and the rhizosphere microbial community structure was analyzed. The results showed that compared with the control group, the cucumber yield was increased by 18.3%, the rhizosphere nitrogenase activity was increased by 16.4%, and the plant nitrogen absorption was increased by 14.8% in the composite microbial agent 1 treatment group. The rhizosphere microbial community structure was analyzed, and the results were shown in Table 10. Compared with the control group, the abundance of *Actinomyces* was increased by 6.4%, the abundance of *Streptomyces* was increased by 13.9%, the abundance of *Rhizobium* was increased by 10.3%, the abundance of *Mesorhizobium* was increased by 8.8%, and the abundance of *Micromonospora* was increased by 10% in the rhizosphere soil of the treatment group. The cucumber yield was increased by 20.9%, the rhizosphere nitrogenase activity was increased by 18.1%, and the nitrogen absorption by the plant was increased by 15.9% in the composite microbial agent 2 treatment group. The rhizosphere microbial community structure was analyzed, and the results were shown in Table 11. Compared with the control group, the abundance of *Actinomyces* was increased by 5.6%, the abundance of *Streptomyces* was increased by 15.7%, the abundance of *Rhizobium* was increased by 11.4%, the abundance of *Mesorhizobium* was increased by 9.7%, and the abundance of *Micromonospora* was increased by 11.2% in the rhizosphere soil of the treatment group.

**[Table 10] Effects of composite microbial agent containing Streptomyces on cucumber yield and rhizosphere microbial community structure**

| | Original soil | Control group | Streptomyces composite microbial agent 1 treatment group | Streptomyces composite microbial agent 2 treatment group |
|---|---|---|---|---|
| Yield (Kg) | - | 287.34 | 339.81 | 347.53 |
| Nitrogenase activity (U/g) | - | 354.71 | 412.81 | 418.76 |
| Nitrogen absorption (Kg) | - | 10.17 | 11.67 | 11.79 |
| Abundance of *Actinomyces* | 1.22% | 1.24% | 1.32% | 1.31% |
| Abundance of *Streptomyces* | 1.08% | 1.08% | 1.23% | 1.25% |
| Abundance of *Rhizobium* | 0.77% | 0.78% | 0.86% | 0.87% |
| Abundance of *Mesorhizobium* | 1.12% | 1.13% | 1.23% | 1.24% |
| Abundance of *Micromonospora* | 0.79% | 0.80% | 0.88% | 0.89% |

### Example 8. Effects of a biopesticide containing Streptomyces on corn yield and rhizosphere microbial community structure

The biopesticide containing *Streptomyces linyiensis* was prepared by mixing *Streptomyces linyiensis* fermentation broth with biopesticides such as thiamethoxam and avermectin. The biopesticide was applied to corn plants to verify the effect. A batch of corn field experiments were arranged and divided into two groups, namely a control group and a treatment group. The control group was fertilized with isonutrient fertilizers, and the treatment group was fertilized with the biopesticide containing *Streptomyces linyiensis.* After 120 days of growth, the yield was measured, rhizosphere soil was collected, soil metagenome was measured, and the rhizosphere microbial community structure was analyzed. The results showed that compared with the control group, the corn yield was increased by 25.6%, the rhizosphere nitrogenase activity was increased by 25.7%, and the plant nitrogen absorption was increased by 14.7% in the treatment group. The rhizosphere microbial community structure was analyzed, and the results were shown in Table 11. Compared with the control group, the abundance of *Actinomyces* was increased by 4.3%, the abundance of *Streptomyces* was increased by 7.6%, the abundance of *Rhizobium* was increased by 10.3%, the abundance of *Mesorhizobium* was increased by 12.1%, and the abundance of *Luteimonas* was increased by 6.5% in the rhizosphere soil of the treatment group.

**[Table 11] Effects of biopesticide containing Streptomyces linyiensis on corn yield and rhizosphere microbial community structure**

| | Original soil | Control group | Streptomyces treatment group |
|---|---|---|---|
| Yield (Kg) | - | 238.69 | 299.75 |
| Nitrogenase activity (U/g) | - | 385.26 | 484.09 |
| Nitrogen absorption (Kg) | - | 10.42 | 11.95 |
| Abundance of *Actinomyces* | 1.15% | 1.16% | 1.21% |
| Abundance of *Streptomyces* | 1.02% | 1.05% | 1.13% |
| Abundance of *Rhizobium* | 0.67% | 0.68% | 0.75% |
| Abundance of *Mesorhizobium* | 0.87% | 0.91% | 1.02% |
| Abundance of *Luteimonas* | 1.06% | 1.07% | 1.14% |

### Example 9. Effects of a biopesticide containing Streptomyces on soybean yield and rhizosphere microbial community structure

The biopesticide containing *Streptomyces linyiensis* was prepared by mixing the Streptomyces powder with biopesticides such as thiamethoxam and avermectin. The biopesticide was applied to soybean plants to verify the effect. A batch of soybean field experiments were arranged and divided into two groups, namely a control group and a treatment group. The control group was fertilized with isonutrient fertilizers, and the treatment group was fertilized with the biopesticide containing *Streptomyces linyiensis.* After 160 days of growth, the yield was measured, rhizosphere soil was collected, soil metagenome was measured, and the rhizosphere microbial community structure was analyzed. The results showed that compared with the control group, the soybean yield was increased by 5.9%, the rhizosphere nitrogenase activity was increased by 14.0%, and the plant nitrogen absorption was increased by 8.2% in the treatment group. The rhizosphere microbial community structure was analyzed, and the results were shown in Table 12. Compared with the control group, the abundance of *Actinomyces* was increased by 4.4%, the abundance of *Streptomyces* was increased by 8.3%, the abundance of *Rhizobium* was increased by 13.8%, the abundance of *Mesorhizobium* was increased by 10.4%, and the abundance of *Luteimonas* was increased by 12.9% in the rhizosphere soil of the treatment group.

**[Table 12] Effects of biopesticide containing Streptomyces linyiensis on soybean yield and rhizosphere microbial community structure**

| | Original soil | Control group | Streptomyces treatment group |
|---|---|---|---|
| Yield (Kg) | - | 123.41 | 130.81 |
| Nitrogenase activity (U/g) | - | 346.57 | 395.09 |
| Nitrogen absorption (Kg) | - | 5.49 | 5.94 |
| Abundance of *Actinomyces* | 0.89% | 0.91% | 0.95% |
| Abundance of *Streptomyces* | 0.96% | 0.96% | 1.04% |
| Abundance of *Rhizobium* | 1.09% | 1.09% | 1.24% |
| Abundance of *Mesorhizobium* | 1.05% | 1.06% | 1.17% |
| Abundance of *Luteimonas* | 0.97% | 1.01% | 1.14% |

In summary, the Streptomyces according to the present disclosure can effectively increase the abundance of nitrogen-fixing microbial strains in the rhizosphere soil of crops, for example, it can increase the abundance of *Actinomyces, Streptomyces, Rhizobium, Mesorhizobium*, *Micromonospora*, or *Luteimonas.* The provided Streptomyces can be prepared into a microbial agent and used as a microbial fertilizer or added to a microbial fertilizer to increase the abundance of the nitrogen-fixing microorganisms in the rhizosphere soil of the crops, increase the nitrogenase activity in the rhizosphere soil of the crops, improve the soil microecological environment, promote the growth of the crops, and increase the yield of the crops.

In the specification, the description of the reference terms such as "one embodiment", "some embodiments", "example", "specific example", or "some examples" means that the specific features, structures, materials, or characteristics described with reference to the embodiment or example are included in at least an embodiment or example of the present disclosure. In this specification, exemplary descriptions of the foregoing terms do not necessarily refer to the same embodiment or example. Moreover, the specific features, structures, materials, or characteristics described may be combined in any one or more embodiments or examples in a suitable manner. Furthermore, those skilled in the art may combine and integrate different embodiments or examples and features of different embodiments or examples described in this specification, unless they are contradictory to each other.

Although embodiments of the present disclosure are illustrated and described above, it can be understood that the above embodiments are illustrative and should not be construed as limitations of the present disclosure. Those skilled in the art can make changes, modifications, substitutions, and variations to the above embodiments within the scope of the present disclosure.

## Claims

1. A microorganism, the microorganism being *Streptomyces linyiensis* deposited on September 28, 2022 in China General Microbiological Culture Collection Center, CGMCC, under CGMCC accession No. 25834.

2. The microorganism according to claim 1, wherein the microorganism has the 16S rDNA sequence as set forth in SEQ ID NO: 1.

3. A fermentation broth, the fermentation broth being obtained by fermenting with the microorganism according to claim 1 or 2.

4. A microbial agent, comprising:
the microorganism according to claim 1 or 2, or
the fermentation broth according to claim 3.

5. The microbial agent according to claim 4, wherein:
the microbial agent is in the form of a dry powder; and
a viable bacterial count of *Streptomyces linyiensis* in the microbial agent is at least 2×10⁹ CFU per gram of the microbial agent.

6. The microbial agent according to claim 4, wherein the microbial agent is obtained by:
fermenting and culturing the *Streptomyces linyiensis* to obtain a fermentation product; and
performing spray-drying and pulverizing treatment on the fermentation product to obtain the microbial agent.

7. A composite microbial agent, comprising the microbial agent according to any one of claims 4 to 6.

8. The composite microbial agent according to claim 7, comprising:
a first microbial agent, being the microbial agent according to any one of claims 4 to 6; and
a second microbial agent, comprising at least one of *Bacillus subtilis*, *Bacillus amyloliquefaciens*, *Bacillus mucilaginosus*, *Streptomyces fradiae*, or *Streptomyces microflavus.*

9. The composite microbial agent according to claim 8, comprising:
10 to 50 parts by weight of the first microbial agent; and
5 to 20 parts by weight of the second microbial agent, wherein the second microbial agent comprises 0 to 5 parts by weight of *Streptomyces microflavus,* 0 to 10 parts by weight of *Bacillus mucilaginosus,* 0 to 5 parts by weight of *Bacillus subtilis,* 0 to 5 parts by weight of *Bacillus amyloliquefaciens*, and 0 to 5 parts by weight of *Streptomyces fradiae.*

10. The composite microbial agent according to claim 8 or 9, wherein in the composite microbial agent, a viable bacterial count of *Streptomyces linyiensis* is at least 2×10⁹ CFU per gram of the composite microbial agent, and wherein at least one of the following conditions is satisfied:
a viable bacterial count of *Streptomyces microflavus* is at least 5×10⁸ CFU per gram of the composite microbial agent;
a viable bacterial count of *Bacillus mucilaginosus* is at least 5×10⁸ CFU per gram of the composite microbial agent;
a viable bacterial count of *Bacillus subtilis* is at least 5×10⁸ CFU per gram of the composite microbial agent;
a viable bacterial count of *Bacillus amyloliquefaciens* is at least 5×10⁸ CFU per gram of the composite microbial agent; or
a viable bacterial count of *Streptomyces fradiae* is at least 5×10⁸ CFU per gram of the composite microbial agent.

11. Use of the microorganism according to claim 1 or 2, the fermentation broth according to claim 3, the microbial agent according to any one of claims 4 to 6, or the composite microbial agent according to any one of claims 7 to 10 in the preparation of a microbial fertilizer or a biopesticide.

12. A microbial fertilizer, comprising:
the microorganism according to claim 1 or 2;
the fermentation broth according to claim 3;
the microbial agent according to any one of claims 4 to 6; or
the composite microbial agent according to any one of claims 7 to 10.

13. The microbial fertilizer according to claim 12, wherein the microbial fertilizer is a solid fertilizer or a liquid fertilizer.

14. The microbial fertilizer according to claim 13, wherein the microbial fertilizer is a solid fertilizer, and wherein a viable bacterial count of the microorganisms contained in the microbial fertilizer is at least 2×10⁹ CFU per gram of the microbial fertilizer.

15. The microbial fertilizer according to claim 13, wherein the microbial fertilizer is a liquid fertilizer, and wherein a viable bacterial count of the microorganisms contained in the microbial fertilizer is at least 2×10⁹ CFU per liter of the microbial fertilizer.

16. The microbial fertilizer according to any one of claims 12 to 15, further comprising a base fertilizer, the base fertilizer comprising at least one of a compound fertilizer, a water-soluble fertilizer, an organic fertilizer, or a foliar fertilizer.

17. A biopesticide, comprising:
the microorganism according to claim 1 or 2;
the fermentation broth according to claim 3;
the microbial agent according to any one of claims 4 to 6; or
the composite microbial agent according to any one of claims 7 to 10.

18. The biopesticide according to claim 17, wherein a viable bacterial count of the microorganisms contained in the biopesticide is at least 2×10⁹ CFU per liter.

19. Use of the microorganism according to claim 1 or 2, the fermentation broth according to claim 3, the microbial agent according to any one of claims 4 to 6, the composite microbial agent according to any one of claims 7 to 10, the microbial fertilizer according to any one of claims 12 to 16, or the biopesticide according to claim 17 or 18 in the increase of abundance of a nitrogen-fixing microorganism in rhizosphere soil of a crop, or the increase of nitrogenase activity in the rhizosphere soil of the crop.

20. The use according to claim 19, wherein the nitrogen-fixing microorganism comprises at least one of *Actinomyces*, *Streptomyces*, *Rhizobium*, *Mesorhizobium*, *Micromonospora*, or *Luteimonas.*

21. A method for increasing abundance of a nitrogen-fixing microorganism in rhizosphere soil of crops, comprising:
applying the microorganism according to claim 1 or 2, the fermentation broth according to claim 3, the microbial agent according to any one of claims 4 to 6, the composite microbial agent according to any one of claims 7 to 10, the microbial fertilizer according to any one of claims 12 to 16, or the biopesticide according to claim 17 or 18 to the crops.

22. The method according to claim 21, wherein the crops comprise at least one of corn, wheat, soybean, rice, potato, sorghum, cucumber, pepper, tomato, peanut, eggplant, or cotton.

23. A method for increasing nitrogenase activity in rhizosphere soil of crops, comprising:
applying the microorganism according to claim 1 or 2, the fermentation broth according to claim 3, the microbial agent according to any one of claims 4 to 6, the composite agent according to any one of claims 7 to 10, the microbial fertilizer according to any one of claims 12 to 16, or the biopesticide according to claim 17 or 18 to the crops.

24. The method according to claim 23, wherein the crops comprise at least one of corn, wheat, soybean, rice, potato, sorghum, cucumber, pepper, tomato, peanut, eggplant, or cotton.
